(19) Europäisches Patentamt
European Patent Office
Office européen des brevets

(11) Publication number: **0 146 900**
**A2**

(12) # EUROPEAN PATENT APPLICATION

(21) Application number: 84115552.6

(22) Date of filing: 15.12.84

(51) Int. Cl.[4]: C 07 D 205/08
C 07 D 405/04

(30) Priority: 27.12.83 JP 251626/83

(43) Date of publication of application:
03.07.85 Bulletin 85/27

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Maeda, Yoshiharu
1757-47 Handa Sayama-cho
Minamikawachi-gun Osaka 589(JP)

(72) Inventor: Kawai, Tatsuhiko
50-1, Yamadaminami
Suita Osaka 565(JP)

(72) Inventor: Sugimoto, Keiichi
11-10, Daiwahigashi 1-chome
Kawanishi Osaka 666-01(JP)

(74) Representative: von Kreisler, Alek, Dipl.-Chem. et al,
Deichmannhaus am Hauptbahnhof
D-5000 Köln 1(DE)

(54) **Process for producing azetidinones.**

(57) An azetidinone of the formula:

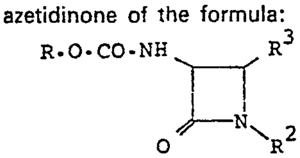

wherein R.O.CO is a protective group for the amino group; $R^2$ is a protective group for the nitrogen atom of the imino compound; $R^3$ is an organic residue bonding through a carbon atom thereof, which is produced with industrial advantages by reacting a compound of the formula:

$$R.O.CO.NH.CH_2.CO.O.CO.OR^1$$

wherein R.O.CO is as defined above; $CO.OR^1$ is an esterified carboxyl group, with a compound of the formula:

$$R^2.N{=}CH.R^3$$

wherein the symbols are as defined above, or a salt thereof.

EP 0 146 900 A2

## Process for producing azetidinones

This invention relates to an industrially advantageous process for producing azetidinones of the formula:

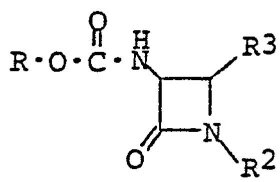

(I)

wherein $R\cdot O\cdot \overset{O}{\overset{\|}{C}}$ is a protective group for the amino group; $R^2$ is a protective group for the nitrogen atom of the imino compound; $R^3$ is an organic residue bonding through a carbon atom thereof, which are of great value as an intermediate for the synthesis of naturally occurring compounds and pharmaceuticals, particularly β-lactam antibiotics.

There have heretofore been known a variety of processes for producing azetidinones, and among such processes is known a cycloaddition reaction as a procedure of producing optically active azetidinone compounds. As the process for producing azetidinones having $R\cdot O\cdot \overset{O}{\overset{\|}{C}}\cdot NH-$ wherein $R\cdot O\cdot \overset{O}{\overset{\|}{C}}$ is as defined hereinbefore at the 3-position by means of the cycloaddition reaction, there are known a process [Journal of Chemical Society, 1880(1975)] involving the reaction of an acid chloride with an imine compound, a method [Synthesis, 210 (1979)] consisting of the reaction of an active ester formed from 2-chloro-N-methylpyridinium iodide and an acid with a Schiff base, and a process [Synthesis, 689 (1976)] comprising

the reaction of an active ester formed from a phosphonium salt and an acid with a Schiff base. These processes, however, suffer from the disadvantages of lowered yield, prolonged reaction time, complicated post-treatment and costly raw materials required, etc. Moreover, it is considered difficult to produce azetidinones, particularly azetidinones having $R\cdot O\cdot \overset{O}{\overset{\|}{C}}\cdot \overset{H}{N}-$ at the 3-position, in one step in satisfactory yields by means of these processes. Therefore, azetidinones having $R\cdot O\cdot \overset{O}{\overset{\|}{C}}\cdot \overset{H}{N}-$ at the 3-position are synthesized (The Japanese Unexamined Patent Publication No. 46060/1983) by reacting a reactive derivative of a compound of the formula $R''-CH_2COOH$ wherein R" is azide, phthalimido or a group represented by $R'OCO-CH=C(CH_3)-NH-$ (wherein R' is lower alkyl), with a Schiff base, then converting the 3-substituent R" of the resulting azetidinones to the amino group and introducing $R\cdot O\cdot \overset{O}{\overset{\|}{C}}$ into the 3-amino group, but this synthetic process, because of an increased number of steps involved, is not industrially advantageous. Since the protective group $R\cdot O\cdot \overset{O}{\overset{\|}{C}}$ for the amino group can allow selection of the type of R through the employed method of deprotecting, on the other hand, azetidinones having $R\cdot O\cdot \overset{O}{\overset{\|}{C}}\cdot \overset{H}{N}-$ at the 3-position are considered of value as a useful intermediate, and an industrially favored production process for such compounds is needed.

The present inventors, under these circumstances as described above, conducted extensive investigation into a method of synthesizing azetidinones having $R\cdot O\cdot \overset{O}{\overset{\|}{C}}\cdot \overset{H}{N}-$ at the 3-position, and as a result, found that a compound of the formula:

$$R\cdot O\cdot \overset{O}{\overset{\|}{C}}\cdot \overset{H}{N}\cdot CH_2\cdot \overset{O}{\overset{\|}{C}}\cdot O\cdot \overset{O}{\overset{\|}{C}}\cdot OR^1 \qquad (II)$$

wherein $R\cdot O\cdot \overset{O}{\overset{\|}{C}}$ is as defined hereinbefore; $\overset{O}{\overset{\|}{C}}\cdot OR^1$ is an esterified carboxyl group can be reacted with a compound of the formula:

$$R^2\cdot N{=}CH\cdot R^3 \qquad (III)$$

wherein the symbols are as defined hereinbefore or a salt thereof to produce the azetidinones (I) in one step and at reduced costs and furthermore that a reaction mixture formed by the reaction of an amino-protected glycine, $R\cdot O\cdot \overset{O}{\overset{\|}{C}}\cdot \overset{H}{N}\cdot CH_2\cdot COOH$ wherein $R\cdot O\cdot \overset{O}{\overset{\|}{C}}$ is as defined hereinbefore, or a salt thereof with an alkyl halogenocarbonate in the presence of a base can be reacted with the compound (III) or a salt thereof to produce the azetidinone (I) in one step and economically, whereby in addition to this, the reaction provides the advantages as an industrial production process, such as shortened reaction time, simplified post-treatment and increased yields. These findings have culminated in the present invention.

Thus, the present invention relates to;

(1) A process for producing the azetidinone (I), characterized in that said process comprises reacting the compound (II) with the compound (III) or a salt thereof, and

(2) A production process as described in the above (1), characterized in that said process comprises reacting a reaction mixture, formed by the reaction of an amino-protected glycine or a salt thereof with an alkyl halogenocarbonate in the presence of a base, with the compound (III) or a salt thereof.

In the above formulas $R\cdot O\cdot \overset{O}{\overset{\|}{C}}$ is a protective group

for the amino group. The amino protecting group represented by $R\cdot O\cdot \overset{O}{\overset{\|}{C}}$ may be conventional ones which are easily removable by conventional means, with those removable by catalytic reduction (especially under catalysis of Pd-C) being particularly preferable. Therefore, R may be, for example, hydrocarbon groups which may be substituted. Examples of such hydrocarbon groups include $C_{1-6}$ alkyl, such as methyl, ethyl, propyl, isopropyl, butyl, isobutyl, sec-butyl and tert-butyl, $C_{6-10}$ aryl, such as phenyl and naphthyl, and $C_{7-11}$ aralkyl, such as benzyl, 1-phenylethyl and naphthl-methyl. These hydrocarbon groups may have 1 to 5 substituents and, when they have not less than two substituents, may have the same or different substituents. Such substituents in the hydrocarbon groups include, for example, halogen, such as chlorine and bromine; cyano; nitro; $C_{1-4}$ alkoxy, such as methoxy and ethoxy; and tri-($C_{1-4}$ alkyl)silyl, such as trimethylsilyl and triethylsilyl. As preferred examples of the amino protecting group represented by $R\cdot O\cdot \overset{O}{\overset{\|}{C}}$, there may be used $C_{7-11}$ aralkyloxycarbonyl, such as benzyloxycarbonyl.

In the above formula, $\overset{O}{\overset{\|}{C}}\cdot OR^1$ is an esterified carboxyl group. Accordingly, $R^1$ is an ester residue, and may be, for example, the hydrocarbon groups as exemplified for R of the above $R\cdot O\cdot \overset{O}{\overset{\|}{C}}$. As preferred examples of $R^1$, there may be used $C_{1-4}$ alkyl, such as methyl, ethyl, propyl, isopropyl, butyl and isobutyl.

$R^2$ is a protective group for the nitrogen atom of the imino compound, in other words, a conventional protective group for the imino group. The protective group for the nitrogen atom of the imino compound as represented by $R^2$ may be, for example, those known as the protective group for the 1-nitrogen atom of azetidinones, or may be protective

group which are easy to be removed. Examples of such imino protecting groups include silyl groups, such as tri-($C_{1-4}$ alkyl)silyl exemplified by tert-butyldimethylsilyl and isopropyldimethylsilyl; $C_{7-11}$ aralkyl, such as benzyl, 1-phenylethyl and naphthylmethyl; di- or tri-($C_{6-10}$ aryl)-methyl, such as benzhydryl and trityl; mono- or di-($C_{1-4}$ alkoxy)-$C_{7-11}$ aralkyl(wherein aralkyl is preferably benzyl), such as 4-methoxybenzyl, and 2,3-, 2,4- or 3,4-dimethoxy-benzyl; mono- or di-($C_{1-4}$ alkoxy)-$C_{6-10}$ aryl(wherein aryl is preferably phenyl), such as 2-, 3- or 4-methoxyphenyl and 2,3-, 2,4- or 3,4-dimethoxyphenyl; di-[mono- or di-($C_{1-4}$ alkoxy)-$C_{6-10}$ aryl]methyl(wherein aryl is preferably phenyl, such as di-(4-methoxyphenyl)methyl; and $C_{3-6}$ alken-2-yl, such as allyl and methallyl.
As more preferred examples of $R^2$, there may be used 2,4- or 3,4-dimethoxybenzyl, benzyl and allyl.

$R^3$ is an organic residue bonding through a carbon atom thereof. Namely, $R^3$ is a residue derived from an organic compound by removal of one hydrogen atom attached to a carbon atom thereof. The organic residue represented by $R^3$ may be, for example, hydrocarbon or heterocyclic groups which may be substituted. As the hydrocarbon groups, in such a case, there may be used, for example, the hydrocarbon groups as exemplified for the above R. As the heterocyclic group, there may be used, for example, 5- or 6-membered saturated or unsaturated heterocyclic groups containing 1 to 4 hetero atoms, each being selected from oxygen, sulfur and nitrogen atoms, and specific examples of such heterocyclic groups include pyrrolyl, furyl, oxolanyl, dioxolyl, dioxolanyl, thienyl, pyridyl, pyranyl, dioxinyl, dioxanyl, thiazolyl, thiazinyl, oxazolyl, isothiazolyl, imidazolyl, thiadiazolyl, oxadiazolyl, triazolyl and tetrazolyl. These hydrocarbon and heterocyclic groups represented by $R^3$ may have, for example, 1 to 3 substituents, and when they have not less than two substituents, may have the same or different substituents.

As the substituents in the hydrocarbon groups represented by $R^3$, there may be used $C_{1-4}$ alkoxy, such as methoxy and ethoxy; $C_{6-10}$ aryloxy, such as phenoxy; $C_{7-11}$ aralkyloxy, such as benzyloxy and 1-phenylethyloxy; hydroxyl; amino; carboxyl; carbamoyloxy; ($C_{1-4}$ alkoxy)carbonyl, such as methoxycarbonyl and ethoxycarbonyl; sulfo; halogen, such as bromine and chlorine; and $C_{1-4}$ alkylthio, such as methylthio and ethylthio. As the substituents in the heterocyclic groups represented by $R^3$, there may be used, for example, the above-mentioned substituents in the hydrocarbon groups as well as oxo and $C_{1-4}$ alkyl, such as methyl and ethyl. In addition, in case the above-mentioned heterocyclic groups represented by $R^3$ have two substituents, such two substituents may be combined to form a divalent $C_{2-6}$ alkylene group [i.e.$-(CH_2)_{2-6}-$], both ends of which are preferably bonded to one carbon atom of the above-mentioned heterocycle. These alkylene groups are, for example, ethylene, trimethylene, tetramethylene and pentamethylene. As preferred examples of $R^3$, there may be used, for example, (R)-2,2-dimethyl-1,3-dioxolan-4-yl, etc.

In the process of the present invention, the azetidinone (I) is obtained by reacting the compound (II) with the compound (III) or a salt thereof.

The starting compound (II) may be synthesized, for example, by a method involving the reaction of an amino-protected glycine or a salt thereof with an alkyl halogenocarbonate, a process as described in Journal of Chemical Society (J. Chem. Soc.), 1880 (1975) or methods similar thereto. Synthesis of the compound (II) is mentioned in more detail hereinafter. The compound (III) may be synthesized, for example, by the method described in the Japanese Unexamined Patent Publication No. 46066/1983 or methods similar thereto.

The compound (II) may be used after being isolated but may also be employed as a reaction mixture obtained in the production of the compound (II). The compound (III) may

be used either in the free state or in the form of a conventional salt. As salts of the compound (III), there may be used, for example, salts with mineral acids such as hydrochloric acid and sulfuric acid, or salts with organic acids such as acetic acid, para-toluenesulfonic acid and oxalic acid. Salts with mineral acids are more preferable in the present invention. This reaction is carried out by allowing 1 mole of the compound (III) or a salt thereof to react with 1 to 6 equivalent moles, preferably 1 to 3 equivalent moles, of the compound (II).

This reaction may be conducted in a solvent. As such a solvent, there may be used, for example, aprotic solvents. Specific examples of the solvent may be ethers, such as tetrahydrofuran and diethyl ether, halogenated hydrocarbons, such as dichloroethane, methylene chloride and chloroform, nitriles, such as acetonitrile, and amides, such as N,N-dimethylformamide and N,N-dimethylacetamide. As the preferred solvent, there may be used, for example, halogenated hydrocarbons such as methylene chloride. In cases in which a reaction mixture obtained in the production of the compound (II) is used as the compound (II), it is advantageous in some instances to carry out this reaction in the said reaction mixture without any solvent employed. The amount of the solvent to be used may be 1 to 30 mℓ per g of the compound (II), preferable 1.5 to 10 mℓ. Also, it is possible to add a base to the reaction system, unless it adversely affects the reaction. As such a base, there may be used, for example, tri-($C_{1-4}$ alkyl)amines, such as trimethylamine, triethylamine and tributylamine. The amount of the base to be used may be 0.01 to 6 moles per mole of the compound (II), preferable 0.1 to 1 mole. As the reaction temperature, there may employed temperatures of -70 to 100°C, preferably -20 to 40°C. The reaction proceeds and completes within a very short period of time. Therefore, the reaction time of up to 2 hours is sufficient, and the preferred reaction time may be 5 to 40 minutes.

After the completion of the reaction, it is also possible to determine the amount of the objective compound (I) produced, for example, by employing high-speed liquid chromatography, etc. The objective compound (I) thus obtained can be isolated and purified by the known isolation methods, such as extraction, washing, chromatography, crystallization and recrystallization, but can also be used, without being isolated, as a starting material in the subsequent reaction.

In the process of the present invention which involves the reaction of the above-described compounds (II) and (III) or a salt thereof to produce the objective compound (I), it is advantageous to use, as the compound (II), a reaction mixture obtained by the reaction of an amino-protected glycine, $R\cdot O\cdot \overset{O}{\overset{\|}{C}}\cdot \overset{H}{N}\cdot CH_2COOH$ or a salt thereof with an alkyl halogenocarbonate in the presence of a base.

As the salt of the amino-protected glycine, there may be used, for example, salts with alkali metals such as sodium and potassium, or salts with tertiary amines such as tri-($C_{1-4}$ alkyl)amine being exemplified by trimethylamine, triethylamine and tributylamine. Preferred examples of the salt of the amino-protected glycine may be, for example, salts with triethylamine and tributylamine.

As the halogen in the alkyl halogenocarbonate, there may be used, for example, bromine and chlorine. As the alkyl in the alkyl halogenocarbonate, there may be used, for example $C_{1-6}$ alkyl, which includes specifically such alkyls as exemplified in the above for R. Preferred examples of the alkyl halogenocarbonate may be, for example, ethyl chlorocarbonate, isopropyl chlorocarbonate and isobutyl chlorocarbonate.

In the case of such a reaction mixture being used, the amount of the amino-protected glycine or a salt thereof to be used may be 1 to 6 equivalent moles per mole of the compound (III), preferably 2 to 4 equivalent moles. The amount of the

alkyl halogenocarbonate to be used may be 1 to 10 equivalent moles per mole of the amino-protected glycine or a salt thereof, preferably 2 to 6 equivalent moles. The reaction of an amino-protected glycine or a salt thereof with an alkyl halogenocarbonate may be carried out in a solvent. As the solvent, there may be used, for example, the solvents as exemplified for the reaction between the compounds (II)and (III) or a salt thereof. The amount of the solvent to be used is 1 to 30 mℓ per g of the amino-protected glycine or a salt thereof, preferably to 5 to 15 mℓ. It is advantageous to carry out this reaction in the presence of a base. Such a base may be, for example, tertiary amines such as tri-($C_{1-4}$ alkyl)amines being exemplified by triethylamine and tributylamine. The amount of the base to be used may be 1 to 7 equivalent moles per mole of the amino-protected glycine or a salt thereof, preferably 2 to 6 equivalent moles. An amino-protected glycine or a salt thereof may be reacted with an alkyl halogenocarbonate in the presence of such a base at a temperature of, for example, -80 to 20°C, preferably -40°C to -10°C. The reaction time may be an extremely short length of time. Concretely, it suffices to allow the reaction to proceed only for a length of time of less than 2 hours, preferably 3 to 10 minutes. The reaction mixture thus obtained is used as such in the subsequent reaction with the compound (III) or a salt thereof. The compound (III) or a salt thereof may be added to the reaction mixture obtained after the above reaction, but may also be added in advance of, or in the course of, the above reaction. For example, such a compound, after being mixed with at least one out of the amino-protected glycine, or a salt thereof, alkyl halogenocarbonate, base and solvent, may be added to the above-mentioned reaction system.

The azetidinone (I) thus obtained can exist in four isomers having cis and trans configurations relative to the

3- and 4-substituents. The present invention is especially suitable and advantageous for producing selectively the (3S, 4S) azetidinones (I), particularly the compounds of the formula:

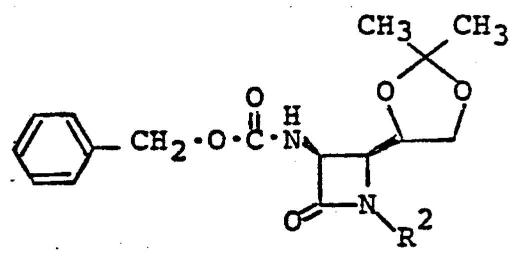

(I')

wherein $R^2$ is as defined hereinbefore. The compounds of the formula (I') can permit the substituents at the 1 and 4 positions to be changed while retaining the 3-substituent (i.e. benzyloxycarboxamido), as will be described hereinafter, and can find widened application as an intermediate and are useful.

The objective compound (I) including (I') thus obtained are the compounds which are of great value as an intermediate for the synthesis of naturally occurring compounds and pharmaceuticals, particularly β-lactam antibiotics, and so forth. With reference to the compounds (I') being representative of the objective compounds (I), for example, the compound of the formula (I') wherein $R^2$ is for example 2,4-dimethoxybenzyl (hereinafter referred to briefly as "DMB") can be converted into (3S,4S)-3-[2-(2-amino-4-thiazolyl)-2-(Z)-(carboxymethoxyimino)acetamido]-4-carbamoyloxymethyl-2-azetidinone-1-sulfonic or a phramaceutically acceptable salt thereof which exihibits excellent antimicrobial activity and the like, for example, by the methods as shown in the following reaction schema (the Japanese Unexamined Patent Publication Nos. 46066/1983 and 189176/1983).

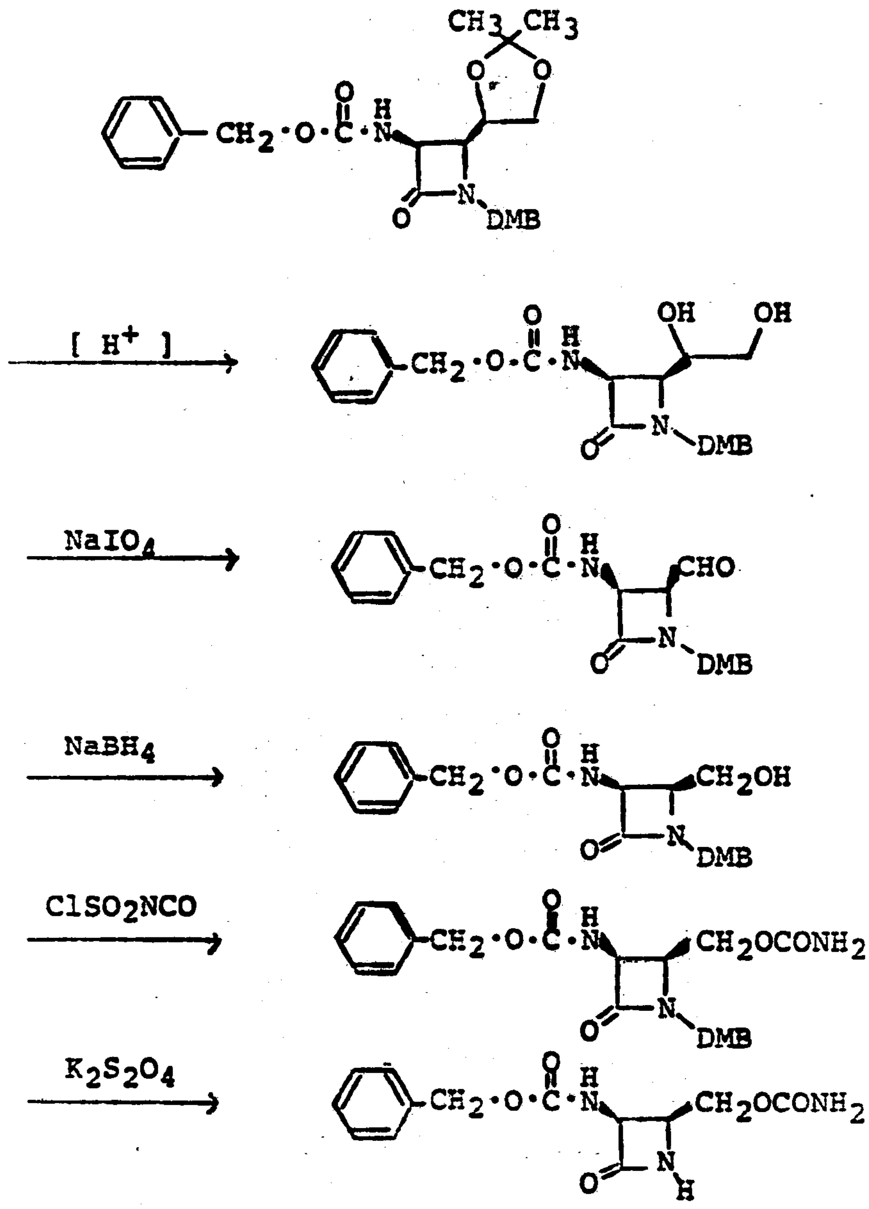
[ H+ ]
NaIO4
NaBH4
ClSO2NCO
K2S2O4

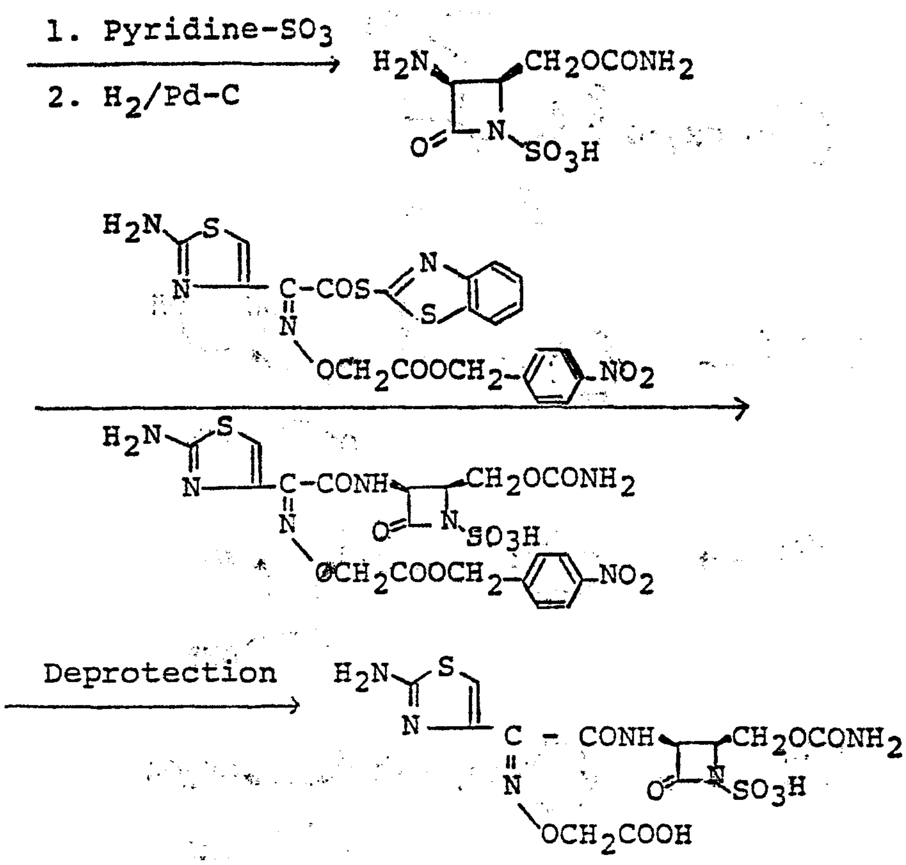


While the present invention is illustrated in detail in the following examples, it is to be understood that these examples are mere embodiments and shall not limit the present invention and that changes and modifications may be made in the present invention without departing from the scope thereof. In the examples, the NMR spectra were measured with an R 24 (60 MHz) type spectrometer (manufactured by Hitachi, Ltd.) using tetramethylsilane as internal or external standard, with the $\delta$ values being shown in ppm. Also, the symbols in the examples have the following meanings:

g: Gram
mℓ: Milliliter
s: Singlet
m: Multiplet
d: Doublet
ϕ: Phenyl
J: Coupling constant
arom.: Aromatic ring

Example 1

In 60 mℓ of methylene chloride is suspended 6.27 g of N-carbobenzoxyglycine, and 6.06 g of triethylamine is added to the suspension to dissolve the N-carbobenzoxyglycine. The resulting solution is cooled to -20°C, and 7.36 g of isopropyl chlorocarbonate is added to it. After allowing the reaction to proceed for 5 minutes, a solution of 2.79 g of (S)-glyceraldehyde acetonide 2,4-dimethoxybenzylimine and 1.52 g of triethylamine in 10 mℓ of methylene chloride is added to the reaction solution.

After allowing the reaction to proceed at room temperature (15 to 30°C) for 30 minutes, the reaction solution is washed with 50 mℓ of water. The organic layer is washed with 50 mℓ of dilute hydrochloric acid and 50 mℓ of water successively. The organic layer is dried over anhydrous magnesium sulfate, and the methylene chloride is distilled off under reduced pressure. The residue is purified by silica-gel column chromatography with use of 180 g of silica gel (elution is effected with a mixed solution of ethyl acetate-n-hexane (1 : 4)) to give 4.0 g (yield of 84.9 %) of crystals of (3S,4S)-3-benzyloxycarboxamido-1-(2,4-dimethoxybenzyl)-4-[(R)-2,2-dimethyl-1,3-dioxolan-4-yl]-2-azetidinone.

IR $\nu_{max}^{KBr}$ $cm^{-1}$: 3310, 1770, 1725, 1695, 1615, 1590, 1536.

$^1$H-NMR (60Mhz, $CDCl_3$)δ: 1.32, 1.42(6H,Sx2,$CH_3$x2), 3.4-3.6 (2H,m,CH-$CH_2$-O), 3.8(6H,S,$OCH_3$x2), 3.8-4.2(4H,m, CH-CH-CH-O,N-CH-arom.), 4.8(1H,d,J=14Hz,N-CH-arom.), 4.95-5.05(1H,m,NH-CH), 5.08(2H,S,ϕ-$CH_2$), 6.0(1H,d, 9Hz,NH), 6.3-6.5(2H,m,arom.), 7.0-7.2(1H,m,arom.), 7.33(5H,S,ϕ).

$[\alpha]_D^{20.5}$ = +45.0°(c=0.2, methanol)

Example 2

In 60 mℓ of methylene chloride is suspended 6.27 g of N-carbobenzoxyglycine, and 6.06 g of triethylamine is added to the suspension to dissolve the N-carbobenzoxyglycine. The solution is cooled to -20°C, and 8.2 g of isobutyl chlorocarbonate is added to it. After allowing the reaction to

proceed for 5 minutes, a solution of 2.79 g of (S)-glyceraldehyde acetonide 2,4-dimethoxybenzylimine and 1.52 g of triethylamine in 10 mℓ of methylene chloride is added to the reaction solution.
After allowing the reaction to proceed at room temperature for 30 minutes, the reaction solution is washed with 50 mℓ of water. The organic layer is washed with 50 mℓ of dilute hydrochloric acid and 50 mℓ of water successively.
The organic layer is dried over anhydrous magnesium sulfate, and the methylene chloride is distilled off under reduced pressure. The residue is dissolved in 5 mℓ of methylene chloride, and 50 mℓ of n-hexane is added to the solution to allow crystals to separate out. The crystals are recovered by filtration and washed with 15 mℓ of a mixed solution of methylene chloride-n-hexane (1 : 4) to give 4.26 g (yield of 90.4 %) of crystals of (3S,4S)-3-benzyloxycarboxamido-1-(2,4-dimethoxybenzyl)-4-[(R)-2,2-dimethyl-1,3-dioxolan-4-yl]-2-azetidinone.
The crystals demonstrate the same IR and NMR spectra as the objective compound of Example 1 does.

Example 3

In 400 mℓ of methylene chloride is suspended 41.8 g of N-carbobenzoxyglycine, and 60.7 g of triethylamine is added to the suspension to dissolve the N-carbobenzoxyglycine. The solution is cooled to -20°C and 65.1 g of ethyl chlorocarbonate is added to it.
After allowing the reaction to proceed for 5 minutes, a solution of 27.9 g of (S)-glyceraldehyde acetonide 2,4-dimethoxybenzylimine and 15.2 g of triethylamine in 150 mℓ of methylene chloride is added to the reaction solution.
After allowing the reaction to proceed at room temperature (15 to 30°C) for 30 minutes, the reaction solution is washed with 500 mℓ of water.
Furthermore, the organic layer is washed with 500 mℓ of dilute hydrochloric acid and then washed with 500 mℓ of water.
The organic layer is dried over anhydrous magnesium sulfate

and the solvent is distilled off under reduced pressure. The residue is dissolved in 50 mℓ of methylene chloride and 500 mℓ of n-hexane is added to the solution to allow crystals to separate out. The crystals are recovered by filtration and washed with 150 mℓ of a mixed solution of methylene chloride n-hexane (1 : 4) to give 38.0 g (yield of 81 %) of crystals of (3S,4S)-3-benzyloxycarboxamido-1-(2,4-dimethoxybenzyl)-4-[(R)-2,2-dimethyl-1,3-dioxolan-4-yl]-2-azetidinone. The crystals demonstrate the same IR and NMR spectra as recorded in Example 1.

Example 4

In 50 mℓ of methylene chloride is suspended 6.3 g of N-carbobenzoxyglycine, and 11.1 g of tributylamine is added to the suspension to dissolve the N-carbobenzoxyglycine. The solution is cooled to -20°C and 6.5 g of ethyl chlorocarbonate is added to it. After allowing the reaction to proceed for 30 minutes, a solution of 2.79 g of (S)-glyceraldehyde acetonide 2,4-dimethoxybenzylimine and 2.8 g of tributylamine in 15 mℓ of methylene chloride is added to the reaction solution. After allowing the reaction to proceed at room temperature (15 to 30°C) for 60 minutes, the reaction solution is washed with 50 mℓ of water. Furthermore, the organic layer is washed with 50 mℓ of dilute hydrochloric acid and 50 mℓ of water successively. The organic layer is dried over anhydrous magnesium sulfate, and the solvent is distilled off under reduced pressure. The residual oily material is dissolved in 5 mℓ of methylene chloride, and 50 mℓ of diisopropyl ether is added to the solution to allow crystals to separate out. The crystals which separate out are recovered by filtration, and washed with diisopropyl ether to give 3.81 g (yield of 81 %) of crystals of (3S,4S)-3-benzyloxycarboxamido-1-(2,4-dimethoxybenzyl)-4-[(R)-2,2-dimethyl-1,3-dioxolan-4-yl]-2-azetidinone. The crystals demonstrate the same IR and NMR spectra as the objective compound of Example 1 does.

## Example 5

In 50 mℓ of methylene chloride is suspended 6.3 g of N-carbobenzoxyglycine, and 6.1 g of triethylamine is added to the suspension to dissolve the N-carbobenzoxyglycine. The solution is cooled to -20°C, and 6.5 g of ethyl chlorocarbonate is added to it. After allowing the reaction to proceed for 30 minutes, a solution of 2.19 g of (S)-glyceraldehyde acetonide 2,4-dimethoxybenzylimine and 1.5 g of triethylamine in 15 mℓ of methylene chloride is added to the reaction solution, followed by reaction at 20°C for 60 minutes. The reaction solution is washed with 50 mℓ of water, and washed further with 50 mℓ of dilute hydrochloric acid.
After the reaction solution is washed with another 50 mℓ of water, the organic layer is dried over anhydrous magnesium sulfate, and the solvent is distilled off under reduced pressure. The residual oily material is dissolved in 5 mℓ of methylene chloride, and 50 mℓ of n-hexane is added to the solution to allow crystals to separate out. The crystals are recovered by filtration and washed with 15 mℓ of a mixed solution of methylene chloride-n-hexane (1 : 1) to give 3.45 g (yield of 84 %) of crystals of (3S,4S)-3-benzyloxycarboxamido-1-benzyl-4-[(R)-2,2-dimethyl-1,3-dioxolan-4-yl]-2-azetidinone.

IR $\nu_{max}^{KBr}$ $cm^{-1}$: 3300, 1756, 1695, 1535.

$^1$H-NMR (60MHz, $CDCl_3$)δ : 1.30, 1.39(6H,Sx2,$CH_3$x2), 3.4-3.6 (2H,m,CH-$CH_2$O), 3.8-4.2(4H,m,CH-CH-CHO,N-CH-arom.), 4.8(1H,d,N-CH-arom.), 4.9-5.1(1H,m,NH-CH), 5.1(2H,S, ϕ-$CH_2$), 6.0(1H,d,NH), 7.2(5H,S,ϕ), 7.4(5H,S,ϕ).

$[\alpha]_D^{25}$ = +40.3° (c=0.3, methanol)

## Example 6

In 250 mℓ of methylene chloride is suspended 25.1 g of N-carbobenzoxyglycine, and 24.2 g of triethylamine is added to the suspension to dissolve the N-carbobenzoxyglycine. The solution is cooled to -20°C and 26.0 g of ethyl chlorocarbonate is added to it. After allowing the reaction to proceed for 5 minutes, a solution of 6.76 g of (S)-glyceraldehyde acetonide allylimine and 6.07 g of triethylamine in 10 mℓ of methylene

chloride is added to the reaction solution. After allowing the reaction to proceed at 40°C for 30 minutes, the reaction solution is washed with 50 mℓ of water and then washed with 50 mℓ of dilute hydrochloric acid. After the reaction solution is further washed with 50 mℓ of water, the organic layer is dried over anhydrous magnesium sulfate, and the methylene chloride is distilled off under reduced pressure. The residue is purified by silica gel column chromatography with use of 500 g of silica gel (elution is effected with a mixed solution of ethyl acetate-n-hexane (1 : 4)) to give 13.6 g (yield of 94.3 %) of crystals of (3S,4S)-3-benzyloxycarboxamido-1-allyl-4-[(R)-2,2-dimethyl-1,3-dioxolan-4-yl]-2-azetidinone.

IR $\nu_{max}^{KBr}$ $cm^{-1}$: 3310, 1760, 1695, 1525.

$^1$H-NMR (60MHz, $CDCl_3$)δ : 1,28, 1.38(6H,Sx2,$CH_3$x2), 3.2-4.5 (7H,m,CH-CH-CH-O,CH-$CH_2$-O,N-$\underline{CH_2}$-CH=$CH_2$), 4.8-5.3(2H, m,-CH=$\underline{CH_2}$), 5.05(2H,S,$\phi CH_2$-), 5.4-6.9(1H,m, $\underline{CH}$=$CH_2$), 6.4(1H,d,J=9Hz), 7.3(5H,S,$\phi$).

$[\alpha]_D^{25.0}$ = +64.3° (c=0.18, methanol)

Example 7

In 350 mℓ of methylene chloride is suspended 37.4 g of N-carbobenzoxyglycine, and 54.3 g of triethylamine is added to the suspension to dissolve the N-carbobenzoxyglycine. The resulting solution is cooled to -20°C, and 58.3 g of ethyl chlorocarbonate is added. After allowing the reaction to proceed for 5 minutes, a solution of 25.0 g of (S)-glyceraldehyde acetonide 3,4-dimethoxybenzylimine and 13.5 g of triethylamine in 60 mℓ of methylene chloride is added to the reaction solution. After allowing the reaction to proceed at room temperature for 30 minutes, the reaction solution is washed with 300 mℓ of water. The organic layer is washed with 300 mℓ of dilute hydrochloric acid and 300 mℓ of water successively. The organic layer is dried over anhydrous magnesium sulfate, and the methylene chloride is distilled off under reduced pressure. The residue is dissolved in 60 mℓ of methylene

chloride and 600mℓ of n-hexane is added to the solution to allow crystals to separate out. The crystals are collected by filtration and washed with 90 mℓ of a mixed solvent of methylene chloride-n-hexane(1:4) to give 32.8 g (yield of 77.9%) of crystals of (3S,4S)-3-benzyloxycarboxamido-1-(3,4-dimethoxybenzyl)-4-[(R)-2,2-dimethyl-1,3-dioxolan-4-yl]-2-azetidinone.

IR$\nu_{max}^{KBr}$ $cm^{-1}$: 3300, 1760, 1690, 1515.

$^{1}H$ NMR(90MHz, $d_6$-DMSO)$\delta$ : 1.23,1.26 (6H,sX2,$CH_3$X2),3.3-4.6(6H,m,CH-CH-$CH_2$O, N-$CH_2$-arom.),3.70,3.72(6H,sX2,$OCH_3$X2), 4.85-9.05(1H,m,-NH-C<u>H</u>-),5.03(2H,s, ø$CH_2$-),6.7-7.0(3H,m,arom.),7.30(5H,s, ø),8.01(1H,d,NH).

$[\alpha]_D^{20}$ = +39.8° (c = 1.02, dimethylsulfoxide)

What we claim is;

1. A process for producing an azetidinone of the formula:

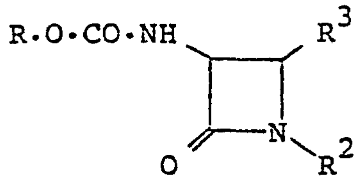

wherein R·O·CO is a protective group for the amino group; $R^2$ is a protective group for the nitrogen atom of the imino compound; $R^3$ is an organic residue bonding through a carbon atom thereof, which comprises reacting a compound of the formula:

$$R \cdot O \cdot CO \cdot NH \cdot CH_2 \cdot CO \cdot O \cdot CO \cdot OR^1$$

wherein R·O·CO is as defined above; $CO \cdot OR^1$ is an esterified carboxyl group, with a compound of the formula:

$$R^2 \cdot N{=}CH \cdot R^3$$

wherein the symbols are as defined above, or a salt thereof.

2. A process as claimed in Claim 1, wherein R is $C_{6-10}$ aryl or $C_{7-11}$ aralkyl.

3. A process as claimed in Claim 1, wherein R is benzyl.

4. A process as claimed in Claim 1, wherein $R^1$ is $C_{1-4}$ alkyl.

5. A process as claimed in Claim 1, wherein $R^2$ is tri-($C_{1-4}$ alkyl)silyl, $C_{7-11}$ aralkyl, di-($C_{6-10}$ aryl)methyl, tri-($C_{6-10}$ aryl)methyl, mono-($C_{1-4}$ alkoxy)-$C_{7-11}$ aralkyl, di-($C_{1-4}$ alkoxy)-$C_{7-11}$ aralkyl, mono-($C_{1-4}$ alkoxy)-$C_{6-10}$ aryl, di-($C_{1-4}$ alkoxy)-$C_{6-10}$ aryl, di-[mono-($C_{1-4}$ alkoxy)-$C_{6-10}$ aryl]methyl, di-[di-($C_{1-4}$ alkoxy)-$C_{6-10}$ aryl]methyl or $C_{3-6}$ alken-2-yl.

6. A process as claimed in Claim 1, wherein $R^2$ is benzyl or dimethoxybenzyl.

7. A process as claimed in Claim 1, wherein $R^2$ is 2,4-dimethoxybenzyl.

8. A process as claimed in Claim 1, wherein $R^2$ is 3,4-dimethoxybenzyl.

9. A process as claimed in Claim 1, wherein $R^3$ is $C_{6-10}$ aryl, $C_{7-11}$ aralkyl or 5- or 6-membered heterocyclic group

containing 1 to 4 hetero atoms each selected among oxygen, sulfur and nitrogen atoms.

10. A process as claimed in Claim 1, wherein $R^3$ is (R)-2,2-dimethyl-1,3-dioxolan-4-yl.

11. A process as claimed in Claim 1, wherein R is benzyl, $R^1$ is ethyl, $R^2$ is 2,4-dimethoxybenzyl and $R^3$ is (R)-2,2-dimethyl-1,3-dioxolan-4-yl.

12. A process for producing an azetidinone of the formula:

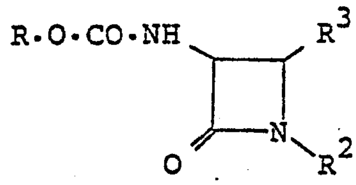

wherein R·O·CO is a protective group for the amino group; $R^2$ is a protective group for the nitrogen atom of the imino compound; $R^3$ is an organic residue bonding through a carbon atom thereof which comprises reacting a reaction mixture, formed by the reaction of an amino-protected glycine or a salt thereof with an alkyl halogenocarbonate in the presence of a base with a compound of the formula:

$$R^2 \cdot N{=}CH \cdot R^3$$

wherein the symbols are as defined above.

13. A process as claimed in Claim 12, wherein the amino-protected glycine is N-carbobenzoxyglycine.